# EUROPEAN PATENT APPLICATION

(11) **EP 4 268 760 A1**
(43) Date of publication of application: **01.11.2023**
(21) Application number: 22170572.6
(22) Date of filing: 28.04.2022
(51) Int. Cl.: A61C 5/62, A46B 11/00, A61C 9/00, B01F 33/501, B05C 17/005, B05C 17/01, A61M 5/315

(54) **MULTI-USE DISPENSER AND APPLICATION SET WITH SUCH A MULTI-USE DISPENSER AND AT LEAST ONE APPLICATOR**

(71) Applicant: medmix Switzerland AG, 9469 Haag (Rheintal) (CH)
(72) Inventor: MÜLLER, Frank, Feldkirch (AT); MOSER, Josef, Unterkulm (CH); NAGARAJA, Dilip Kumar, Karnataka (IN); ABUNDIS DE HILBE, Vanessa, Triesenberg (LI)
(74) Representative: Manitz Finsterwald Patent- und Rechtsanwaltspartnerschaft mbB

(57) **Abstract**

The present invention refers to a multi-use dispenser 1 for use with an applicator 11 in particular for dental applications. The multi-use dispenser 1 comprises a main body 3 forming a housing and a dispense mechanism 5 provided at least partly within the main body 3. The dispense mechanism 5 comprises a piston rod 7 axially movable with respect to the main body 3 in a proximal direction for a dispense operation. The main body 3 comprises connection means 13 configured to couple an applicator 11 to the multi-use dispenser 1 in a releasable manner such that a proximal movement of the piston rod 7 results in a proximal movement of a piston 33 for substance dispense. The dispense mechanism 5 comprises a dispense activator 17 configured to be operated by a user and to move the piston rod 7 a specific predefined distance in the proximal direction upon operation. Furthermore, the present inventio refers to an application set comprising such a multi-use dispenser and at least one, in particular at least two, corresponding applicators.

## Description

The present invention relates to a multi-use dispenser for use with an applicator, in particular for dental applications, and to an application set comprising such a multi-use dispenser and at least one corresponding applicator.

To date, syringe like dispensers are known for the application of substances from applicators. Such configurations allow a highly flexible setting of the amount to be dispensed by varying the insertion depth of the piston rod. However, it has been observed that often such a dispense operation without any restrictions or even feedback often results in an amount of dispensed substance not corresponding to the intended amount. Often, too much substance is dispensed, in particular due to relaxation effects after stopping the movement of the piston rod resulting in the dispense of more substance than intended. On the other hand, if the user is aware of such a problem, the user is often too careful during dispense often resulting in a probably problematic (due to premature drying) hesitant and/or incomplete dispense of the substance.

In view of this, it is an object of the present invention to provide a multi-use dispenser allowing a reliable and exact dispense of substance from an applicator.

This object is solved by the multi-use dispenser of claim 1. Preferable modifications for such a multi-use dispenser are given in the dependent claims. Furthermore, the present invention refers to an application set comprising such a multi-use dispenser and at least one corresponding applicator, as well as to various preferable modifications for such an application set.

According to the present invention, a multi-use dispenser for use with an applicator, in particular for dental applications, comprises a main body forming a housing and a dispense mechanism provided at least partly within the main body. The dispense mechanism comprises a piston rod, in particular with a piston pusher, axially movable with respect to the main body in a proximal direction for a dispense operation. The main body comprises, in particular keyed, connection means, in particular in the form of Luer-Lock or bayonet connection means, configured to couple an applicator to the multi-use dispenser in a releasable manner such that a proximal movement of the piston rod results in a dispense operation of the applicator. The dispense mechanism comprises a dispense activator, in particular comprising a dispense knob, configured to be operated by a user and to move the piston rod a specific predefined distance in the proximal direction upon operation.

The term multi-use dispenser in the sense of the present invention refers not to a dispenser configured to multiply dispense substance from one single applicator but to a dispenser configured to dispense substances from multiple applicators releasably coupled to the dispenser. Nevertheless, the present understanding of the term multi-use dispenser also includes configurations in which multiple doses of substances can be dispensed with the dispenser from each of the multiple applicators.

With the dispense activator of the present configuration resulting in the predefined movement of the piston rod upon each operation, a predefined amount of substance from the applicator is dispensed per operation of the dispense activator reliably and exactly. The predefined distance moved by the piston rod can in particular take into account any relaxation effects. Thus, the user can dispense reliably and exactly a specific amount of the substance from the applicators or if desired a multiple of this specific amount by a multiple operation of the dispense activator from a single applicator.

In this connection it should be noted that the dispenser can be configured for use with a plethora of substances. Such materials can range from adhesives, filler materials, cleaning materials, lubricants, fluids, liquids, such as pharmaceutical liquids, whiteners, and ointments, used in the dental, medical, pharmaceutical and veterinary fields, to adhesives, lubricants and paints used in industrial applications, e.g. in the automotive, and aeronautical fields, and in the construction sector etc. Preferably, the dispense activator is configured to be operated in a direction perpendicular to the axial dispense direction of the piston rod.

Such a configuration results in an improved handling of the multi-use dispenser and further allows the implementation of highly compact configurations.

Preferably, the dispense activator is coupled operatively to the piston rod via an activation spring member fixed to the dispense activator and in engagement with the piston rod.

Such a configuration is structurally quite simple and, thus, cost efficient but still reliable.

Preferably, the dispense mechanism comprises a resetting spring biasing the dispense activator from its operated position into its non-operated position.

Such a configuration results in a further improved handling of the multi-use dispenser and, in particular, enables an easy multi dispense operation with a single applicator.

Preferably, the dispense mechanism comprises a rear body axially movable together with the piston rod along the main body and configured to support the piston rod in its orientation with respect to the main body.

Such a configuration is very robust and, thus, also reliable.

Preferably, the dispense activator comprises a brake element contacting the piston rod in a non-operated position of the dispense activator to lock an axial movement of the piston rod.

Such a configuration reduces the risk of an undesired axial movement of the piston rod with the dispense activator not being operated.

Preferably, the piston rod comprises a toothed configuration that is engaged by a clicker member resulting in a haptic and/or acoustic feedback during a proximal movement of the piston rod.

Such a configuration allows a user a more exact dispense operation with the multi-use dispenser and a further specified operation of the dispense operator by only partially operating the dispense activator if desired.

Preferably, the dispense mechanism comprises a piston rod resetting configuration allowing the piston rod to be reset after a dispense operation with a first applicator for a further dispense operation with a second applicator by retracting the piston rod back in the distal direction opposing the proximal direction.

Such a configuration allows the easy resetting of the multi-use dispenser for the usage with the second applicator after a dispense operation with the first applicator. Such a resetting configuration can be manually or automatically operated. In automatic configurations, the resetting movement of the piston rod can be triggered by a decoupling of the first applicator from the multi-use dispenser or can result from a coupling of the second applicator to the multi-use dispenser.

Further preferably, the piston rod resetting configuration is configured such that the piston rod can be turned by 90° about its longitudinal axis, in which position the piston rod can be retracted.

Such a resetting configuration is quite robust and reliably allows a very quick resetting of the piston rod.

In particular, the piston rod resetting configuration comprises a smooth circumferential section of the piston rod.

Such an implementation is quite simple but reliable.

Alternatively, the piston rod resetting configuration is configured such that the piston rod can be retracted by a screwing movement of the piston rod.

Such a configuration is very robust and, thus, highly reliable.

In particular the piston rod resetting mechanism is formed by a thread about the circumference of the piston rod.

Such an implementation is quite simple and allows a very robust overall configuration.

A further aspect of the present invention refers to an application set comprising one of the above described multi-use dispensers and at least one, in particular at least two, applicator(s) for such a multi-use dispenser, in particular for dental applications. Each applicator comprises a cartridge body defining a reservoir filled with an, in particular dentistry applicable, substance. The substances from different applicators can be the same or can differ from each other. Each cartridge body is connected to a dispense body with a dispense opening through which the substance from the corresponding reservoir can be dispensed. A piston is provided movably along the corresponding reservoir within each cartridge body and is configured to dispense the substance from the corresponding reservoir upon movement in a proximal direction towards the dispense body. Each applicator comprises, in particular keyed, connection means, in particular in the form of Luer-Lock or bayonet connection means, configured to couple the respective applicator to the connection means of the multi-use dispenser in a releasable manner, such that the piston is movable in the proximal direction by a dispense operation of the multi-use dispenser.

Such a configuration is quite simple and, thus, cost efficient, while allowing the easy use of the multi-use dispenser with various applicators.

Preferably, the dispense body of each applicator comprises a distribution means at, in particular around, the dispense opening.

Such a distribution means allows an improved application and, in particular, a homogeneous distribution of the substance.

In particular the distribution means comprises a brush configuration at, in particular around, the dispense opening. Preferably the brush configuration is formed as a micro toothbrush.

Such an implementation is quite simple but highly effective.

Alternatively, the distribution means comprises a foam configuration at, in particular around, the dispense opening.

Such an implementation depicts a simple and also highly effective alternative to the above implementation.

Preferably, the cartridge body and the dispense body, and in particular also the distribution means, of each applicator are formed from a single material.

Such a configuration allows a direct observation of the dispense movement of the piston and, thus, an easy observation of the dispense operation.

Preferably, the cartridge body and the dispense body, and in particular also the distribution means, of each applicator are formed as single one-piece member.

Such a configuration is very robust.

Preferably, each applicator comprises a plug or cap configured to be coupled releasably to the dispense body to seal the dispense opening.

Before and after the dispense operation, the cap allows to seal the dispense opening preventing the leakage of (remaining) substance form the reservoir.

Exemplary embodiments and functions of the present invention are described herein in conjunction with the following drawings, wherein:
- FIG. 1: shows a sectional view of an exemplary multi-use dispenser in accordance with the present invention in a non-operated state and coupled to a schematically illustrated applicator;
- FIG. 2: shows a perspective cross-section of the rear part of the multi-use dispenser of FIG. 1 looking in the distal direction;
- FIG. 3: shows a perspective view of a first exemplary embodiment for an applicator to be used with the multi-use dispenser of the present invention;
- FIG. 4: shows a perspective view of a second exemplary embodiment for an applicator to be used with the multi-use dispenser of the present invention; and
- FIG. 5: shows a perspective view of a third exemplary embodiment for an applicator to be used with the multi-use dispenser of the present invention.

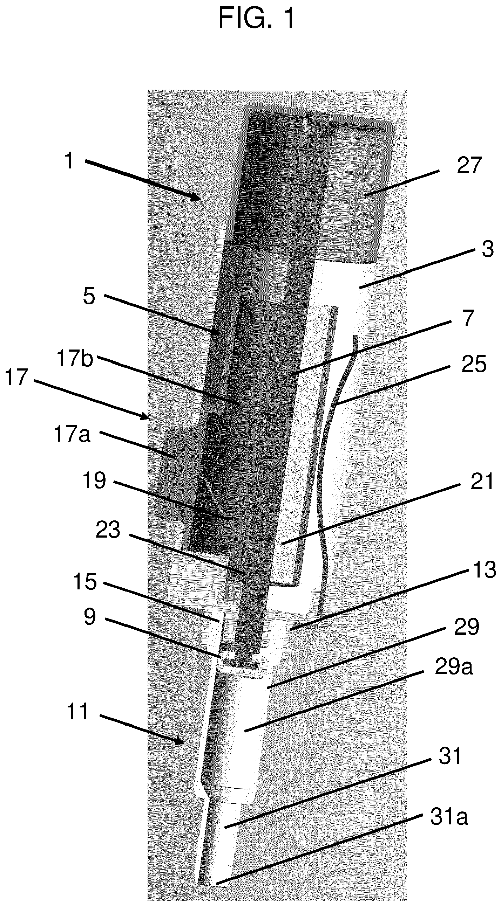

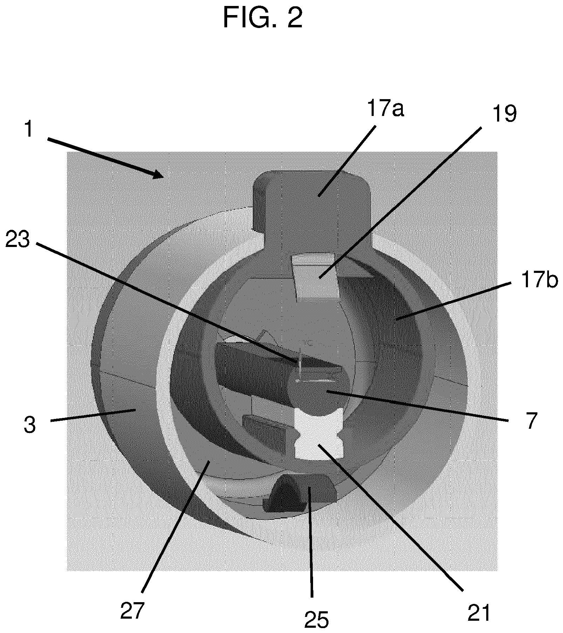

In the following, the structural configuration of a preferred exemplary embodiment of a multi-use dispenser 1 in accordance with the present invention will be described with reference to FIGS. 1 and 2. Its function will be described further below.

As can be seen in particular in FIG. 1 the illustrated multi-use dispenser 1 comprises a main body 3 forming a housing of the multi-use dispenser 1. The main body 3 encloses a dispense mechanism 5. Said dispense mechanism 5 comprises a piston rod 7 axially movable with respect to the main body 3 in a proximal direction (in FIG. 1 downwards in the direction of the applicator 11 described later). The piston rod 7 is provided with a piston pusher 9 for pushing a piston 33 (not illustrated here) of an applicator 11. The main body 3 comprises connection means 13 configured to couple connection means 15 of the applicator 11 to the multi-use dispenser 1 in a releasable manner such that a proximal movement of the piston rod 7 results in a proximal dispense movement of the piston 33 (not illustrated here) of the applicator 11 for dispense. In particular, the connection means 13 are keyed connection means allowing only the coupling of applicators 11 with matching keyed connection means 15 to the multi-use dispenser 1. Preferably, the coupling between the multi-used dispenser 1 and the applicator 11 if formed by Luer-Lock or bayonet connection means.

The dispense mechanism 5 of the multi-use dispenser 1 comprises further a dispense activator 17. Here, the dispense activator 17 comprises a dispense knob 17a to be operated by a user in a direction perpendicular to the axial dispense direction (following the longitudinal axis) of the piston rod 7, and an activator sleeve 17b surrounding the piston rod 7 at least partly.

The dispense activator 17 is coupled operatively to the piston rod 7 via an activation spring member 19 and a brake element 21.

The activation spring member 19 is fixed with a first end thereof to the dispense activator 17 and is in engagement with a toothed configuration 23 of the piston rod 7 with a second end thereof (see FIG. 1). Said toothed configuration 23 is formed by radially extending teeth provided on a certain circumferential section of the piston rod 7 (see FIG. 2). The remaining circumference of the piston rod 7 comprises no such teeth but an in particular smooth surface and an in particular circular cross section. The brake element 21 is coupled fixedly to the activator sleeve 17b and positioned between the activator sleeve 17b and the piston rod 7 in the radial direction of the piston rod 7.

Furthermore, a resetting spring 25 is preferred positioned opposite the dispense knob 17a and configured to bias the dispense activator 17 from its operated position into its into its non-operated position illustrated in FIGS. 1 and 2). However, also other positions for the resetting spring 25 are possible, as long as it is configured to bias the dispense activator 17 in the described manner.

Finally, a rear body 27 is preferred connected fixedly to the rear part of the piston rod 7 and configured to move together with the piston rod 7 along the main body 3 together with the piston rod 7. The read body 27 is provided within the main body 3 in such a manner that it supports the rear part of the piston rod 7 in its orientation with respect to the main body 3, such that the longitudinal axis of the piston rod 7 is always aligned with the longitudinal axis of the main body 3.

As an alternative to the described rear body 27, a rear-cap (not illustrated) can be provided to close the distal end of the main-body 3. In such a configuration, the piston rod 7 can extend through a through-hole within the rear-cap to be supported. Moreover, the piston rod 7 can be connected at its distal end to a moulded element, in particular in the form of a gripping element for the user.

In the following, the function of the illustrated multi-use dispenser 1 will be described.

At first, the multi-use dispenser 1 is in the non-operated state illustrated in FIG. 1 and a matching applicator 11 is coupled via the keyed connection means 13 and 15 to the multi-use dispenser 1. In this state, the dispense activator 17 is biased by the resetting spring 25 in its non-operated position, i.e. to the left in FIG. 1 or to the top in FIG. 2. Consequently, the brake element 21 is squeezed between the activator sleeve 17b and the piston rod 7 fixing the piston rod 7 against axial movement thereof relative to the main body 3.

To dispense a predefined amount of substance from the applicator 11, the user pushes the dispense knob 17a inwardly against the resetting force of the resetting spring 25 and against the activation spring member 19. Thus, the activator sleeve 17b moves together with the dispense knob 17a in a direction perpendicular to the longitudinal axis of the piston rod 7 resulting in a resolving of the contact between the brake member 21 and the piston rod 7. At the same time, the activation spring member 19 abuts with its second end against the teeth of the toothed configuration 23 of the piston rod 7 and is deformed resulting in an axially directed movement of the piston rod 7 towards the applicator 11. This movement results a proximal dispense movement of the piston 33 (not illustrated) of the applicator 11. The toothed configuration 23 of the piston rod 7 is in further engagement with a clicker member (not illustrated) formed at the breakthrough of the piston rod 7 through the main body 3 near the connection means 13, providing a haptic and/or acoustic feedback to a user during the dispense operation.

Upon full activation of the dispense activator 17, the piston rod 7 has moved a predefined distance along the main body 3 determined by the specific geometric configuration of the components of the dispense mechanism 5. In particular, the predefined distance traveled by the piston rod 7 upon operation of the dispense activator 17 is specified by the available movement distance of the dispense activator 17 perpendicular to the piston rod 7 and the shape of the activation spring member 17. This predefined amount predefines the amount of substance dispensed form the applicator 11 upon a single dispense operation caused by a single operation of the dispense activator 17.

After the full operation of the dispense activator 17, the user releases the dispense knob 17a. Thus, the resetting spring 25 moves back the dispense activator 17 into its non-operated position resulting in the brake element 21 contacting and fixing the piston rod 7 again. At the same time, the second end of the activation spring member 19 moves along the teeth of the toothed configuration 23 for moving back into its original shape.

Now, the dispense activator 17 may be operated again for a further dispense operation or the applicator 11 is decoupled from the multi-use dispenser 1. For resetting the piston rod 7 of the multi-use dispenser 1 for usage with a second applicator 11, the dispense activator 17 is operated at least partly for resolving the contact between the brake element 21 and the piston rod 7. Then, the rear body 27 is rotated by at least 90° about the longitudinal axis of the piston rod 7 resulting due to an also rotational coupling between these two components in a rotation of the piston rod 7. This rotational movement results in a disengagement of the activation spring member 19 from the toothed configuration 23 of the piston rod 7. In this state, the user can retract the rear body 27 (or the moulded element at the piston rod's 7 distal end) together with the piston rod 7 into its original relative orientation with respect to the main body 3. This resetting movement can be supported by a further provided spring element (not illustrated), which is compressed by the proximal dispense movement of the piston rod 7. After the full resetting movement, the user turns the rear body 27 (or the moulded element) together with the piston rod 7 back into its original orientation resulting in a re-engagement of the activation spring member 19 with the toothed configuration 23.

Now, a new applicator 11 can be coupled to the multi-use dispenser 1 for a further dispense operation.

Alternatively, to the above described toothed configuration 23, the piston rod 7 can comprise a thread configuration along its outer circumference being in engagement with the activation spring member 19. Such a configuration would allow a screwing retraction movement of the piston rod 7 without decoupling the activation spring member 19 from the piston rod 7.

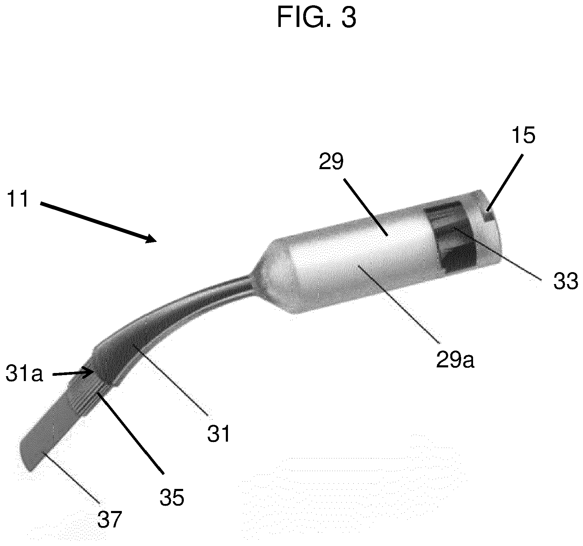

In the following, three preferred exemplary embodiments for applicators 11 for usage with multi-use dispensers 1 in accordance with the present invention and in particular with the above described multi-use dispenser 1 are described with reference to FIGS. 3 to 5. Such applicators 11 are suitable to be combined with the above described multi-use dispenser 1 to form an application set in the sense of the present invention.

Each applicator 11 comprises a cartridge body 29 defining a reservoir 29a filled with an in particular dentistry applicable substance. Each cartridge body 29 is connected to a dispense body 31 with a dispense opening 31a through which the substance from the corresponding reservoir 29a can be dispensed. A piston 33 is provided within and movable along the reservoir 29a to dispense the substance from the reservoir 29a upon movement in a proximal direction towards the dispense body 31 (i.e. to the left in FIGS. 3 to 5). For coupling the applicators 11 in a releasable manner to the multi-use dispenser 1 each applicator 11 comprises the above referred to, in particular keyed, connection means 15. The multi-use dispenser 1 and the applicator 11 are configured such that in the coupled state the piston 33 is movable in the proximal direction by a dispense operation of the multi-use dispenser 1 and in particular by a corresponding proximal movement of the piston rod 7.

In the illustrated embodiments, each dispense body comprises a distribution means 35 at, in particular around, the dispense opening 31a.

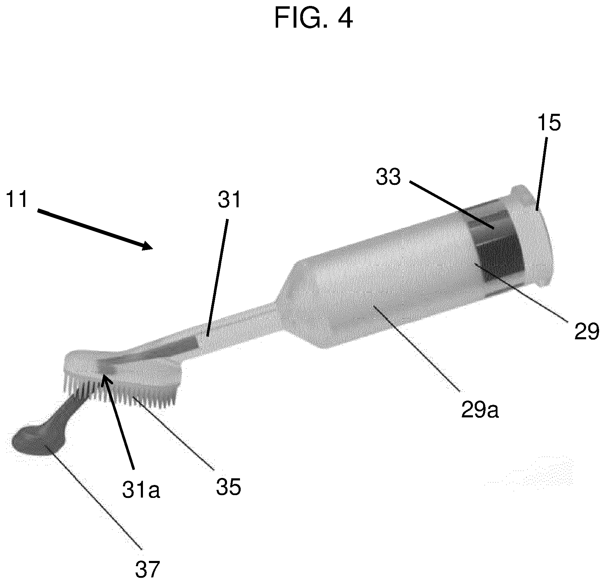

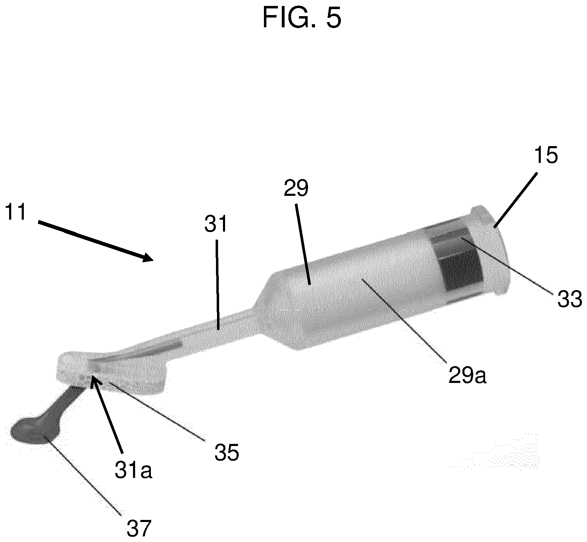

As illustrated in particular in FIG. 3, said distribution means 35 can comprise a brush configuration at, in particular around, the dispense opening 31a. In particular, said brush configuration can be formed as a micro tooth-brush as illustrated in FIG. 4. However, also other implementations for the distribution means 35 like a foam configuration at, in particular around, the dispense opening 31a, as for example illustrated in FIG. 5, are possible.

Preferably, as it is the case in all three illustrated embodiments, the cartridge body 29 and the dispense body 31, and in particular also the distribution means 35, of each applicator 11 is formed from a single, in particular translucent, material and, furthermore or alternatively, as single one-piece member. A translucent material is of particular relevance for applicators with primarily cosmetic dental products. For specific applications, in particular for applicators filled with light-cured sealants or fillings, the material can be only translucent for light of specific wavelengths preventing curing of the substance within the applicator or the material can be non-translucent.

Finally, each applicator 11 comprises a plug or cap 37 configured to be coupled releasably to the dispense body 31 to seal the dispense opening 31a, in particular before and after the dispense operation.

Finally, it is pointed out that the above described exemplary embodiments are just provided for the sake of illustration and not for the sake of delimitation. The scope of protection of the present document is only defined by the accompanied set of claims and covers various modifications not described exemplary in this document.

It should be noted that the terms distal and proximal used in the foregoing relate to the positions of use of the applicator 11, i.e. a proximal direction or movement means a direction towards, e.g. the mouth of a patient (not shown) on use of the applicator 11 and a distal direction or movement refers to a movement away from the patient.

### Reference numeral list

- 1: multi-use dispenser
- 3: main body
- 5: dispense mechanism
- 7: piston rod
- 9: piston pusher
- 11: applicator
- 13: connection means of multi-use dispenser 1
- 15: connection means of applicator 11
- 17: dispense activator
- 17a: dispense knob
- 17b: activator sleeve
- 19: activation spring member
- 21: brake element
- 23: toothed configuration
- 25: resetting spring
- 27: rear body
- 29: cartridge body
- 29a: reservoir
- 31: dispense body
- 31a: dispense opening
- 33: piston
- 35: distribution means
- 37: plug or cap

## Claims

1. A multi-use dispenser (1) for use with an applicator (11) in particular for dental applications,
wherein the multi-use dispenser (1) comprises a main body (3) forming a housing and a dispense mechanism (5) provided at least partly within the main body (3),
wherein the dispense mechanism (5) comprises a piston rod (7), in particular with a piston pusher (9), axially movable with respect to the main body (3) in a proximal direction for a dispense operation,
wherein the main body (3) comprises, in particular keyed, connection means (13), in particular in the form of Luer-Lock or bayonet connection means, configured to couple an applicator (11) to the multi-use dispenser (1) in a releasable manner such that a proximal movement of the piston rod (7) results in a dispense operation via the applicator (11), wherein the dispense mechanism (5) comprises a dispense activator (17), in particular comprising a dispense knob (17a), configured to be operated by a user and to move the piston rod (7) a specific predefined distance in the proximal direction upon operation.

2. The multi-use dispenser (1) of claim 1,
wherein the dispense activator (17) is configured to be operated in a direction perpendicular to the axial dispense direction of the piston rod (7).

3. The multi-use dispenser (1) of claim 1 or 2,
wherein the dispense activator (17) is coupled operatively to the piston rod (7) via an activation spring member (19) fixed to the dispense activator (17) and in engagement with the piston rod (7).

4. The multi-use dispenser (1) of any one of claims 1 to 3,
wherein the dispense mechanism (5) comprises a resetting spring (25) biasing the dispense activator (17) from its operated position into its non-operated position.

5. The multi-use dispenser (1) of any one of claims 1 to 4,
wherein the dispense mechanism (5) comprises a rear body (27) or a moulded element axially movable together with the piston rod (7) along the main body (3) and configured to support the piston rod (7) in its orientation with respect to the main body (3).

6. The multi-use dispenser (1) of any one of claims 1 to 5,
wherein the dispense activator (17) comprises a brake element (21) contacting the piston rod (7) in a non-operated position of the dispense activator (17) to lock an axial movement of the piston rod (7).

7. The multi-use dispenser (1) of any one of claims 1 to 6,
wherein the piston rod (7) comprises a toothed configuration (23) that is engaged by a clicker member resulting in a haptic and/or acoustic feedback during a proximal movement of the piston rod (7).

8. The multi-use dispenser (1) of any one of claims 1 to 7,
wherein the dispense mechanism (5) comprises a piston rod resetting configuration allowing the piston rod (7) to be reset after a dispense operation with a first applicator (11) for a further dispense operation with a second applicator (11) by retracting the piston rod (7) back in the distal direction opposing the proximal direction.

9. The multi-use dispenser (1) of claim 8,
wherein the piston rod resetting configuration is configured such that the piston rod (7) can be turned by 90° about its longitudinal axis, into an orientation in which the piston rod (7) can be retracted,
wherein in particular the piston rod (7) resetting configuration comprises a smooth circumferential section of the piston rod (7).

10. The multi-use dispenser (1) of claim 8,
wherein the piston rod resetting configuration is configured such that the piston rod (7) can be retracted by a screwing movement of the piston rod (7),
wherein in particular the piston rod resetting mechanism is formed by a thread about the circumference of the piston rod (7).

11. An application set comprising a multi-use dispenser (1) of any one of the preceding claims 1 to 10 and at least one, in particular at least two, applicator(s) (11) for such a multi-use dispenser (1), in particular for dental applications,
wherein each applicator (11) comprises a cartridge body (29) defining a reservoir (29a) filled with an in particular dentistry applicable substance, wherein each cartridge body (29) is connected to a dispense body (31) with a dispense opening (31a) through which the substance from the corresponding reservoir (29a) can be dispensed,
wherein a piston (33) is provided movably along the corresponding reservoir (29a) within each cartridge body (29) and is configured to dispense the substance from the corresponding reservoir (29a) upon movement in a proximal direction towards the dispense body (31), wherein each applicator (11) comprises, in particular keyed, connection means (15), in particular in the form of a Luer-Lock or bayonet connection means, configured to couple the respective applicator (11) to the connection means (13) of the multi-use dispenser (1) in a releasable manner, such that the piston (33) is movable in the proximal direction by a dispense operation of the multi-use dispenser (1).

12. The application set of claim 11,
wherein the dispense body (31) of each applicator (11) comprises a distribution means (35) at, in particular around, the dispense opening (31a);
wherein in particular the distribution means (35) comprises a brush configuration at, in particular around, the dispense opening (31a), wherein preferably the brush configuration is formed as a micro tooth-brush; or
wherein the distribution means (35) comprises a foam configuration at, in particular around, the dispense opening (31a).

13. The application set of claim 11 or 12,
wherein the cartridge body (29) and the dispense body (31), and in particular also the distribution means (35), of each applicator (11) are formed from a single material.

14. The application set of any one of claims 11 to 13,
wherein the cartridge body (29) and the dispense body (31), and in particular also the distribution means (35), of each applicator (11) are formed as single one-piece member.

15. The application set of any one of claims 11 to 14,
wherein each applicator (11) comprises a plug or cap (37) configured to be coupled releasably to the dispense body (31) to seal the dispense opening (31a).
